# EUROPEAN PATENT SPECIFICATION

(11) **EP 1 093 337 B1**
(45) Date of publication and mention of the grant of the patent: **09.05.2007**
(21) Application number: 99939286.3
(22) Date of filing: 20.08.1999
(51) Int. Cl.: A23L 1/305, A61K 31/385, A61K 31/195

(54) **FOOD SUPPLEMENTS COMPRISING LIPOIC ACID AND CREATINE AND METHODS FOR THEIR USE**
NAHRUNGSMITTELERGÄNZUNG, WELCHE LIPONSÄURE UND KREATIN ENTHÄLT UND METHODEN ZU DEREN ANWENDUNG
SUPPLEMENTS NUTRITIFS COMPRENANT DE L'ACIDE LIPOIQUE ET DE LA CREATINE ET METHODES POUR LEUR UTILISATION

(30) Priority: 21.08.1998 CA 2246014; 21.08.1998 US 138136
(43) Date of publication of application: 25.04.2001
(73) Proprietor: Muscletech Research and Development Inc., Mississauga, Ontario L5S 1V7 (CA)
(72) Inventor: GARDINER, Paul, T., Brampton, Ontario L6S 2H6 (CA)
(74) Representative: Beetz & Partner
(86) International application number: PCT/CA1999/000772
(87) International publication number: WO 2000/010408

(56) References cited:
- EP-A- 0 702 953
- EP-A- 0 812 590
- NETRITION.COM "MET-RX CREATINE AC" [ONLINE] AVAILABLE FROM INTERNET: <URL:HTTP://WWW.NETRITION.COM/CYTOPRO_PAGE .HTML> 30 NOVEMBER 1999, pages 1-7, XP002124392
- MAXIMUSCLE "THE ULTIMATE 'ALL IN ONE' POWER PRODUCT" [ONLINE] AVAILABLE FROM INTERNET: <URL: WWW.MAXIMUSCLE.COM/CYCLONE.HTML> 01.12.99, XP002124446
- THE NUTRASENSE COMPANY "CREATINE MTS TRANSPORT SYSTEM" [ONLINE] AVAILABLE FROM INTERNET: <URL:WWW.NUTRASENSE.COM/NUTRASENSE/CREATIN EMTS.HTML> 30. NOVEMBER 1999 , XP002124393
- VITANET "MUSCLE TECH'S CELL-TECH" [ONLINE] AVAILABLE FROM INTERNET: <URL:HTTP://STORE.YAHOO.COM/VITANET/MUSTEC CEL4PO.HTML> 30. NOVEMBER 1999, XP002124394
- PHYSICAL ENHANCEMENT "EXTREME SUPPLEMENTATION" [ONLINE] AVAILABLE FROM INTERNET: <URL:HTTP://PHYSICALENHANCEMENT.COM/SUPP/E XTREME4.HTML> 30. NOVEMBER 1999, XP002124395
- "ATHLETENGOLD" [ONLINE] AVAILABLE FROM INTERNET: <HTTP://STORE.YAHOO.COM/ATHLETENGOLD/INLEM ANDGRAP.HTML> 01.DECEMBER 1999, XP002124447

## Description

### FIELD OF INVENTION

The present invention is directed to food supplements which comprise lipoic acid or derivative thereof and creatine or a derivative thereof, and to methods for supplementing the diet of an athlete and methods for enhancing an athlete's muscle size or strength, which methods employ these food supplements.

### BACKGROUND OF INVENTION

Creatine (also known as N-methyl-N-guanyl glycine or (alpha methyl guanido) acetic acid) is an amino acid compound produced naturally in the liver and kidneys and obtained from food such as meat and fish. Food supplements containing creatine, typically creatine monohydrate, are commonly used by athletes to allow them to train harder and enhance muscle size and strength. Various commercial products containing creatine monohydrate are available.

Lipoic acid (also known as alpha-lipoic acid, thioctic acid or 6,8-dithio octanoic acid) is a nutrient that the human body makes in minute quantities and may be obtained from yeast and liver. Studies have shown that lipoic acid can significantly increase the body's utilization of blood sugar in type II diabetics and that lipoic acid may increase the metabolic clearance rate of glucose by 50% in diabetics. In Europe, lipoic acid has been used as a substitute for insulin in the treatment of Type II diabetes.

Food supplements for enhancing an athlete's muscle size and strength have become popular substitutes for steroids and other drugs in various sports and body building regimes. However, as athletes continually strive for improved performance, there is a continuing need for non-steroid containing aids for improving muscle size and strength.

### SUMMARY OF THE INVENTION

Accordingly, it is an object of the present invention to provide new food supplements. It is a more specific object of the invention to provide food supplements particularly adapted for supplementing the diet of an athlete. It is another object to provide food supplements which may enhance an athlete's muscle size or strength. It is a further object of the invention to provide non-steroid containing food supplements and to provide food supplements which may be conveniently administered to the diet of an athlete.

These and additional objects are provided by the food supplements and methods according to the present invention as defined in the claims. The food supplements comprise lipoic acid or a derivative thereof, for example a salt or ester thereof, and creatine or a derivative thereof, for example a hydrate, salt or ester thereof. The food supplements may optionally, and preferably, further comprise dextrose. It has been determined that such food supplement compositions are advantageous for supplementing the diet of an athlete and may provide surprising enhancement of an athlete's muscle size or strength when administered to an athlete's diet. These and additional objects and advantages will be more fully apparent in view of the following detailed description.

### DETAILED DESCRIPTION OF THE INVENTION

The food supplement compositions of the present invention comprise lipoic acid or a derivative thereof and creatine or a derivative thereof. Creatine and hydrates thereof, particularly creatine monohydrate, are known to improve and/or enhance muscle size and/or strength. The food supplements and methods of the present invention may provide further and significant muscle size and strength enhancement or improvement as compared with supplements and methods employing only creatine or a hydrate thereof. Although the present invention is not to be limited by any theoretical explanation, it is believed that insulin is a primary factor that stimulates glucose and creatine transport into the muscle cells and that the lipoic acid both mimics and enhances the actions of insulin in glucose and creatine transport into the muscle cells. In preferred embodiments, the compositions further include dextrose which has been shown to stimulate insulin action.

The food supplements comprise lipoic acid or a derivative thereof, for example a salt or ester thereof. Suitable salts include, but are not limited to, alkali and alkaline earth metal salts, for example sodium, potassium or calcium salts, while suitable esters include, but are not limited to, alkyl esters, for example, methyl, ethyl or propyl esters, or lactone esters.

The food supplement compositions further comprise creatine or a derivative thereof, for example a hydrate, salt or ester thereof. Commercially available creatine derivatives include creatine monohydrate, other creatine hydrates, creatine citrate and creatine pyruvate. The creatine which is employed in the food supplement compositions of the present invention preferably comprises creatine monohydrate, commercially available from various sources. It is similarly preferred that the creatine, creatine monohydrate or other creatine derivative is a pharmaceutical-grade material. As set forth above, the food supplement compositions also preferably further comprise dextrose (glucose), with pharmaceutical-grade dextrose being preferred.

The food supplement compositions of the present invention may be provided in liquid or powder form, with powders suitable for mixing with water or other liquids being preferred. The food supplement compositions in powder or granular form may be provided in accordance with customary processing techniques, for example as spray dried powders, or the like. Owing to the lipoic acid component, the food supplement compositions generally exhibit an acidic pH value.

The lipoic acid or salt, ester or other derivative thereof is employed in the food supplement compositions according to the present invention in an amount sufficient to increase creatine transport into the muscle cells. In a preferred embodiment, the food supplement compositions comprise from about 0.1 mg to about 10 mg lipoic acid or derivative thereof per gram of supplement and from about 0.01 g to about 0.5 g of creatine, preferably creatine monohydrate, or derivative thereof, per gram of supplement. In further preferred embodiments, the food supplement compositions comprise from about 0.5 mg to about 5 mg lipoic acid derivative thereof per gram of supplement and from about 0.05 g to about 0.25 g of creatine or derivative thereof per gram of supplement. Even more preferably the food supplement compositions comprise from about 1 mg to about 3 mg lipoic acid, salt, ester or other derivative thereof per gram of supplement and from about 0.05 g to about 0.2 g of creatine, creatine monohydrate or other derivative thereof per gram of supplement. Food supplement compositions further comprising dextrose preferably include the dextrose in an amount sufficient to stimulate insulin secretion and preferably the food supplement compositions comprise from about 0.1 g to about 0.9 g dextrose per gram of supplement. In further preferred embodiments, the food supplement compositions comprise from about 0.4 g to about 0.9 g dextrose per gram of supplement, and more preferably comprise from about 0.5 g to about 0.8 g dextrose per gram of supplement. Most preferably, the food supplement compositions comprise about 0.75 g dextrose per gram of supplement

The food supplement compositions according to the present invention may further contain additional components to further increase the speed at or ease with which creatine enters the bloodstream and subsequently the muscle tissue, or to otherwise enhance the effects of the creatine in the body. For example, additional amino acids may be included in the food supplement compositions. Suitable amino acids include, but are not limited to, glutamine, alanine, taurine, carnitine, acetyl-L-carnitine, and the like. These additional amino acids may stimulate cell volumization and protein synthesis and therefore provide further advantages to increasing muscle strength and/or size. These amino acids may be employed individually or in various combinations and in amounts customary in the art, for example in the range of from about 0.01 mg to about 100 mg per gram of food supplement.

In a further preferred embodiment, the food supplement compositions also include a chromium compound. Again, while not intending to be limited by theory, various studies have indicated that chromium supplementing will improve glucose tolerance in insulin-sensitive individuals by up to 50% and thereby maximize insulin efficiency. Chromium is a constituent of a biologically active compound, the glucose tolerance factor (GTF), found in foods such as organ meats, whole grains, cheese, mushrooms and brewer's yeast. Various chromium compounds may be included in the food supplement compositions, and in amounts effective to improve insulin efficiency. A preferred chromium compound is chromium picolinate, which may be included, for example in an amount of from about 50 to about 500 micrograms per 100 grams of supplement. Additional components for the food supplement compositions include additional minerals such as magnesium, potassium, phosphorous, salts thereof, or mixtures thereof in amounts conventional in the art, for example, from about 0.01 mg to about 100 mg per gram of food supplement. The food supplement compositions also preferably contain ascorbic acid (vitamin C), for example in amounts equal to or exceeding the recommended minimum daily requirements. Another component for use in the food supplements comprises beta-hydroxy, beta-methyl butyrate (HMB), in amounts known in the art

The food supplement compositions may further comprise natural and/or artificial flavoring components, dyes or other coloring additives, preservatives and other conventional food supplement additives known in the art.

The food supplements according to the present invention may be employed in methods for supplementing the diet of an athlete, and/or for enhancing an athlete's muscle size or strength. The food supplement compositions of the present invention are particularly advantageous for creating an increased anabolic environment and obtaining extra growth in lean muscle mass and strength. The amount of the food supplement composition which is administered to the diet of the athlete may vary depending on the desired effect, the body weight and characteristics of the athlete, and the like. For example, in preferred methods for supplementing the diet of an athlete and/or for enhancing an athlete's muscle size or strength, from about 10 mg to about 1,000 mg of lipoic acid, salt, ester or other derivative thereof and from about 1 g to about 50 g of creatine, creatine monohydrate, or other derivative thereof are administered to the diet of the athlete on a daily basis. In more preferred embodiments of these methods, from about 50 mg to about 500 mg of lipoic acid or derivative thereof and from about 5 g to about 25 g of creatine, creatine monohydrate or other derivative thereof are administered to the diet of the athlete on a daily basis. The food supplement may be administered in a single serving or in multiple servings spaced throughout the day. In alternate preferred embodiments, from about 100 mg to about 300 mg of lipoic acid or derivative thereof and from about 5 g to about 20 g of creatine, creatine monohydrate or other derivative thereof are administered to the diet of the athlete on a daily basis.

To facilitate such administration, preferred embodiments of the food supplement of the invention comprise from about 0.1 mg to about 10 mg lipoic acid or derivative thereof per gram of supplement and from about 0.01 g to about 0.5 g of creatine, creatine monohydrate, or other derivative thereof per gram of supplement, and the supplement is administered in an amount of from about 10 g to about 500 g per day. In additional embodiments, such supplements are administered in an amount of from about 50 g to about 300 g per day, in one or multiple servings throughout the day.

In additional embodiments wherein the supplement further comprises dextrose, from about 10 mg to about 1,000 mg of lipoic acid or derivative thereof, from about 1 g to about 50 g of creatine, creatine monohydrate or other derivative thereof, and from about 10 g to about 300 g of dextrose are administered to the diet of the athlete on a daily basis. In further preferred embodiments, from about 50 mg to about 500 mg of lipoic acid or derivative thereof, from about 5 g to about 25 g of creatine, creatine monohydrate, or other derivative thereof, and from about 50 g to about 200 g of dextrose administered to the diet of the athlete on a daily basis. Most preferably, each supplement serving comprises from about 100 mg to about 300 mg lipoic acid or a derivative thereof, from about 5 g to about 20 g of creatine, creatine monohydrate or other derivative thereof, and from about 50 g to about 90 g, more preferably about 75 g, of dextrose.

When the food supplement further comprises dextrose, a supplement comprising from about 0.1 mg to about 10 mg lipoic acid or derivative thereof per gram of supplement, from about 0.01 g to about 0.5 g of creatine or derivative thereof per gram of supplement, and from about 0.1 g to about 0.9 g dextrose per gram of supplement, preferably from about 0.5 g to about 0.8 g dextrose per gram of supplement, may be administered in an amount of from about 10 g to about 500 g, more preferably from about 50 g to about 300 g, per day.

In one embodiment of the methods according to the present invention, increased servings of the food supplement according to the present invention may be initially administered to the athlete's diet in order to increase or enhance the athlete's muscle size or strength, followed by a maintenance period in which decreased servings of the food supplement may be administered. The initial period may be at least several days and may extend up to several weeks as desired. Once a desired muscle strength and/or size has been obtained, lower servings of the food supplement may be administered to the athlete's diet in order to maintain the increased muscle size and/or strength. These features will be discussed in further detail in the examples. Additionally, in order to maximize the effects of the food supplement in enhancing muscle size and/or strength, it is preferred that the food supplement is administered to the diet of the athlete immediately following an exercise period. On non-workout days, the food supplement may be administered anytime during the day, although administering the food supplement upon awakening or otherwise during the morning hours is preferred.

The food supplement compositions and methods of the invention are further illustrated in the following examples. In the examples and throughout the present specification, parts and percentages are by weight unless otherwise specified.

### EXAMPLES

### EXAMPLE 1

In this example, an athlete consumes two servings of the food supplement as described herein daily, one in the morning and the other twelve hours later or immediately after his/her exercise workout. This regime is continued for five days in order to enhance muscle size and/or strength. Each serving of the food supplement is approximately 100 g and contains the following:

| Component | Amount |
|---|---|
| Lipoic Acid | 200 mg |
| Creatine Monohydrate | 10g |
| Dextrose | 75 g |
| Taurine | 2000 mg |
| Vitamin C | 250mg |
| Magnesium | 70 mg |
| Potassium | 150 mg |
| Phosphorous | 100 mg |
| Chromium Picolinate | 300 mcg |

Each approximate 100 gram serving is mixed with 340g (12 ounces) of cold water to provide a liquid drink. An additional 8 ounces of water is consumed after the food supplement liquid drink is consumed.

### EXAMPLE 2

The muscle size and/or strength enhancing regime of Example 1 is modified so that the athlete consumes four servings of the food supplement daily, with each serving being approximately 50 g of the supplement and comprising:

| Component | Amount |
|---|---|
| Lipoic Acid | 100 mg |
| Creatine Monohydrate | 5 g |
| Dextrose | 37.5 g |
| Taurine | 1000 mg |
| Vitamin C | 125 mg |
| Magnesium | 35 mg |
| Potassium | 75 mg |
| Phosphorous | 50 mg |
| Chromium Picolinate | 150 mcg |

Each supplement serving is mixed with 340 g (12 ounces) of cold water and the servings are administered at approximately evenly spaced times through the day, with one serving being consumed immediately after the athlete's exercise workout.

### EXAMPLE 3

Once the regime described in Example 1 is completed, the athlete continues with a maintenance regime wherein one serving of the food supplement composition described in Example 1 is consumed on a daily basis. The serving is consumed immediately after an exercise workout or, on non-workout days, upon awakening or otherwise in the morning hours. As described in Example 1, the approximate 100 gram serving is mixed with cold water to form a liquid drink. Alternatively, the food supplement is be combined with other liquid drinks or foods as desired. This maintenance regime is continued for at least eight weeks to maintain enhanced muscle size and/or strength.

The servings set forth in these examples are designed for a 2000 calorie diet. Daily values may be increased or decreased depending on the calorie needs of individual athletes, and/or body weights of individual athletes.

The examples and embodiments set forth in the present application are provided only to illustrate various aspects of the invention.

## Claims

1. Food supplement comprising two components A and B, component A being selected from lipoic acid or a derivative thereof, and component B being selected from creatine or a derivative thereof.

2. Food supplement according to claim 1, wherein the creatine or creatine derivative is selected from creatine hydrates, creatine salts or creatine esters.

3. Food supplement according to claim 1 or 2, wherein the creatine or creatine derivative is selected from creatine monohydrate, creatine citrate or creatine pyruvate.

4. Food supplement according to any of claims 1 to 3, further comprising dextrose, ascorbic acid, taurine, magnesium, potassium, phosphorus, chromium, salts thereof, or mixtures thereof.

5. Food supplement according to any of claims 1 to 4, comprising 0.1 mg to 10 mg of lipoic acid or of a derivative thereof per gram of supplement, and 0.01 g to 0.5 g of creatine or of a derivative thereof per gram of supplement.

6. Food supplement according to any of claims 1 to 5, comprising 0.5 mg to 5 mg of lipoic acid or of a derivative thereof per gram of supplement, and 0.05 g to 0.25 g of creatine or of a derivative thereof per gram of supplement.

7. Food supplement according to any of claims 1 to 6, comprising 1 mg to 3 mg of lipoic acid or of a derivative thereof per gram of supplement, and 0.05 g to 0.2 g of creatine or of a derivative thereof per gram of supplement.

8. Food supplement according to any of claims 1 to 7, comprising 0.1 g to 0.9 g, particularly 0.4 g to 0.9 g and, preferably, 0. 5 g to 0.8 g of dextrose per gram of supplement.

9. Food supplement according to any of claims 1 to 8, comprising lipoic acid: 200 mg, creatine monohydrate: 10 g, dextrose: 75 g, taurine: 2000 mg, vitamin C: 250 mg, magnesium: 70 mg, potassium: 150 mg, phosphorus: 100 mg, and chromium picolinate: 300 µg, per 100g of supplement.

10. Food supplement according to any of the claims 1 to 9, comprising: lipoic acid: 100 mg, creatine monohydrate: 5 g, dextrose: 37,5 g, taurine: 1000 mg, vitamin C: 125 mg, magnesium: 35 mg, potassium: 75 mg, phosphorus: 50 mg, and chromium picolinate: 150 µg, per 50g of supplement.

11. Method for supplementing the diet of an athlete, comprising administering to the diet of the athlete the food supplement of any of claims 1 to 10.

12. The method according to claim 11, wherein the food supplement is administered immediately following an exercise period.

13. A method for preparing a food supplement as in any one of claims 1 to 10, comprising the steps of preparing the lipoic acid and creatine in powder or liquid form.

## Patentansprüche

1. Nahrungsergänzungsmittel, das zwei Komponenten A und B enthält, wobei die Komponente A unter Liponsäure oder einem Derivat davon und die Komponente B unter Kreatin oder einem Derivat davon ausgewählt ist.

2. Nahrungsergänzungsmittel nach Anspruch 1, wobei das Kreatin oder das Kreatinderivat unter Kreatinhydraten, Kreatinsalzen oder Kreatinestern ausgewählt ist.

3. Nahrungsergänzungsmittel nach Anspruch 1 oder 2, wobei das Kreatin oder das Kreatinderivat unter Kreatinmonohydrat, Kreatincitrat oder Kreatinpyruvat ausgewählt ist.

4. Nahrungsergänzungsmittel nach einem der Ansprüche 1 bis 3, das ferner Dextrose, Ascorbinsäure, Taurin, Magnesium, Kalium, Phosphor, Chrom, deren Salze oder deren Gemische enthält.

5. Nahrungsergänzungsmittel nach einem der Ansprüche 1 bis 4, das 0,1 bis 10 mg Liponsäure oder ein Derivat davon pro Gramm Nahrungsergänzungsmittel und 0,01 bis 0,5 g Kreatin oder ein Derivat davon pro Gramm Nahrungsergänzungsmittel enthält.

6. Nahrungsergänzungsmittel nach einem der Ansprüche 1 bis 5, das 0,5 bis 5 mg Liponsäure oder ein Derivat davon pro Gramm Nahrungsergänzungsmittel und 0,05 bis 0,25 g Kreatin oder ein Derivat davon pro Gramm Nahrungsergänzungsmittel aufweist.

7. Nahrungsergänzungsmittel nach einem der Ansprüche 1 bis 6, das 1 bis 3 mg Liponsäure oder ein Derivat davon pro Gramm Nahrungsergänzungsmittel und 0,05 bis 0,2 g Kreatin oder ein Derivat davon pro Gramm Nahrungsergänzungsmittel enthält.

8. Nahrungsergänzungsmittel nach einem der Ansprüche 1 bis 7, das 0,1 bis 0,9 g, insbesondere 0,4 bis 0,9 g und vorzugsweise 0,5 bis 0,8 g Dextrose pro Gramm Nahrungsergänzungsmittel enthält.

9. Nahrungsergänzungsmittel nach einem der Ansprüche 1 bis 8, das enthält: Liponsäure: 200 mg, Kreatinmonohydrat: 10 g, Dextrose: 75 g, Taurin: 2 000 mg, Vitamin C: 250 mg, Magnesium: 70 mg, Kalium: 150 mg, Phosphor: 100 mg und Chrompicolinat: 300 µm, pro 100 g Nahrungsergänzungsmittel.

10. Nahrungsergänzungsmittel nach einem der Ansprüche 1 bis 9, das enthält: Liponsäure 100 mg, Kreatinmonohydrat: 5 g, Dextrose: 37,5 g, Taurin: 1 000 mg, Vitamin C: 125 mg, Magnesium: 35 mg, Kalium: 75 mg, Phosphor: 50 mg und Chrompicolinat: 150 µm, pro 50 g Nahrungsergänzungsmittel.

11. Verfahren zur Ergänzung der Ernährung eines Sportlers, das das Verabreichen des Nahrungsergänzungsmittels nach einem der Ansprüche 1 bis 10 zur Ernährung des Sportlers beinhaltet.

12. Verfahren nach Anspruch 11, wobei das Nahrungsergänzungsmittel unmittelbar nach einer Trainingseinheit verabreicht wird.

13. Verfahren zum Herstellen eines Nahrungsergänzungsmittels nach einem der Ansprüche 1 bis 10, das die Schritte der Zubereitung von Liponsäure und Kreatin in Form eines Pulvers oder einer Flüssigkeit umfasst.

## Revendications

1. Supplément nutritif comprenant deux composants A et B, le composant A étant sélectionné parmi l'acide lipoïque ou un de ses dérivés et le composant B étant sélectionné parmi la créatine ou un de ses dérivés.

2. Supplément nutritif selon la revendication 1, dans lequel la créatine ou le dérivé de créatine est sélectionné parmi les hydrates de créatine, les sels de créatine ou les esters de créatine.

3. Supplément nutritif selon la revendication 1 ou 2, dans lequel la créatine ou le dérivé de créatine est sélectionné parmi le monohydrate de créatine, le citrate de créatine ou le pyruvate de créatine.

4. Supplément nutritif selon l'une quelconque des revendications 1 à 3, comprenant en outre le dextrose, l'acide ascorbique, la taurine, le magnésium, le potassium, le phosphore, le chrome, leurs sels ou leurs mélanges.

5. Supplément nutritif selon l'une quelconque des revendications 1 à 4, comprenant 0.1 mg à 10 mg d'acide lipoïque ou un de ses dérivés par gramme de supplément, et 0.01 g à 0.5 g de créatine ou d'un de ses dérivés par gramme de supplément.

6. Supplément nutritif selon l'une quelconque des revendications 1 à 5, comprenant 0.5 mg à 5 mg d'acide lipoïque ou un de ses dérivés par gramme de supplément, et 0.05 g à 0.25 g de créatine ou d'un de ses dérivés par gramme de supplément.

7. Supplément nutritif selon l'une quelconque des revendications 1 à 6, comprenant 1 mg à 3 mg d'acide lipoïque ou un de ses dérivés par gramme de supplément, et 0.05 g à 0.2 g de créatine ou d'un de ses dérivés par gramme de supplément.

8. Supplément nutritif selon l'une quelconque des revendications 1 à 7, comprenant 0.1 g à 0.9 g, en particulier de 0.4 g à 0.9 g et, de préférence 0.5 g à 0.8 g de dextrose par gramme de supplément.

9. Supplément nutritif selon l'une quelconque des revendications 1 à 8, comprenant 200 mg d'acide lipoïque, 10 g de monohydrate de créatine, 75 g de dextrose, 2000 mg de taurine, 250 mg de vitamine C, 70 mg de magnésium, 150 mg de potassium, 100 mg de phosphore et 300 µg de picolinate de chrome pour 100 g de supplément.

10. Supplément nutritif selon l'une quelconque des revendications 1 à 9, comprenant 100 mg d'acide lipoïque, 5 g de monohydrate de créatine, 37.5 g de dextrose, 1000 mg de taurine, 125 mg de vitamine C, 35 mg de magnésium, 75 mg de potassium, 50 mg de phosphore et 150 µg de picolinate de chrome pour 50 g de supplément.

11. Procédé d'utilisation de supplément de régime d'un athlète, comprenant l'introduction dans le régime de l'athlète du supplément nutritif selon l'une quelconque des revendications 1 à 10.

12. Procédé selon la revendication 11, dans lequel le supplément nutritif est administré immédiatement à la suite d'une période d'exercice.

13. Procédé pour la préparation d'un supplément nutritif tel que revendiqué dans l'une quelconque des revendications 1 à 10, comprenant les étapes consistant à préparer l'acide lipoïque et la créatine sous forme de poudre ou de liquide.
